# EUROPEAN PATENT SPECIFICATION

(11) **EP 2 296 583 B1**
(45) Date of publication and mention of the grant of the patent: **13.03.2013**
(21) Application number: 09742107.7
(22) Date of filing: 06.05.2009
(51) Int. Cl.: A61F 2/30, A61L 27/30, A61L 27/46

(54) **DUAL-SIDED JOINT IMPLANT HAVING A WEAR RESISTANT SURFACE AND A BIOACTIVE SURFACE**
DOPPELSEITIGES GELENKIMPLANTAT MIT EINER VERSCHLEISSFESTEN OBERFLÄCHE UND EINER BIOAKTIVEN OBERFLÄCHE
IMPLANT D'ARTICULATION À DEUX CÔTÉS AYANT UNE SURFACE RÉSISTANT À L'USURE ET UNE SURFACE BIOACTIVE

(30) Priority: 06.05.2008 EP 08155701
(43) Date of publication of application: 23.03.2011
(73) Proprietor: Episurf Medical AB, 115 42 Stockholm (SE)
(72) Inventor: NYGREN, Mats, S-167 73 Bromma (SE); XU, Changming, Shanghai 200050 (CN); RYD, Leif, S-113 40 Stockholm (SE); FLODSTRÖM, Katarina, SE-11362 Stockholm (SE); BAKE, Nina, S-181 90 Lidingö (SE)
(74) Representative: Kitzler, Michael
(86) International application number: PCT/EP2009/055506
(87) International publication number: WO 2009/135889

(56) References cited:
- US-A- 4 636 218
- US-A- 5 192 325
- US-A1- 2004 243 241
- US-B1- 6 306 925

## Description

### Field of the invention

The present invention relates generally to an implant device, and more specifically the invention relates to an implant device for an articular surface in a joint such as a knee, elbow or shoulder. The present invention also relates to a method for manufacturing such an implant.

### Background

Pain and overuse disorders of the joints of the body is a common problem. For instance, one of the most important joints which is liable to wearing and disease is the knee. The knee provides support and mobility and is the largest and strongest joint in the body. Pain in the knee can be caused by for example injury, arthritis or infection. The weight-bearing and articulating surfaces of the knees, and of other joints, are covered with a layer of soft tissue that typically comprises a significant amount of hyaline cartilage The friction between the cartilage and the surrounding parts of the joint is very low, which facilitates movement of the joints under high pressure. The cartilage is however prone to damage due to disease, injury or chronic wear. Moreover it does not readily heal after damages, as opposed to other connective tissue, and if healed the durable hyaline cartilage is often replaced by less durable fibrocartilage. This means that damages of the cartilage gradually become worse. Along with injury/disease comes a problem with pain which results in handicap and loss of function. It is therefore important to have efficient means and methods for repairing damaged cartilage in knee joints.

Today's knee prostheses are successful in relieving pain but there is a limit in the lifetime of the prostheses of 10-15 years. The surgical operation is demanding and the convalescence time is often around 6-12 months. In many cases today, surgery is avoided if training and painkillers can reduce the pain. Prostheses are therefore foremost for elderly patients in great pain, at the end of the disease process; a totally destroyed joint. There are different kinds of prostheses, such as half prosthesis, total prosthesis and revision knee, the latter used after a prosthesis failure. The materials used in today's knee prostheses are often a combination of a metal and a polymeric material, but other materials such as ceramics have also been used. The size of knee prostheses makes it necessary to insert them through open surgery.

Smaller implants for replacement of damaged cartilage have also been developed (see e.g. US2003060887, WO2004075777 and US20020022889 recited below in the prior art section). These are however, still rather large and/or require big and robust attachment means to firmly attach the implant to the underlying bone.

Other attempts practiced at various clinics around the world with the main objective to repair or rebuild cartilage include biological approaches such as micro fractures, cartilage cell transplantation (ACI), periost flap, and mosaic plasty surgery. All treatments have shown only limited results, with implications such as high cost, risk of infection, risk of loosening, limited suitability for patients of different ages and the extent and location of damage.

The advantages of implants have stimulated a further development of smaller implants that can be implanted with less invasive surgery. In this development there has also been an effort to achieve small joint implants that have a minimal influence on the surrounding parts of the joint. In addition, better fitting and ideally tailor made implants are desired.

### Prior art

The patent documents US2003060887, US20030171820, US20070255412, US20060116774 and US20020062154 show examples of various implants comprising a bioactive material combined with different types of more inert material.

Examples of prior art concerned with fixation of implant to bone tissue are found in the publications: WO2004075777, WO2005084216 WO2006004885, WO2006091686 US2007179608 and US20020022889.

Examples of prior art appearing on the market can currently be found under the following http links:
http://www.arthrosurface.com
http:/www.conformis.com/
http:www.advbiosurf.com/

The patent document US20070021838 describes joint implants having a bone-contacting side and an articular side, with at least one post extending from the bone contacting side for the purpose of fastening the implant to the bone. The articular side is made of a biocompatible materials such as a medical alloy, medical plastics, ceramics or natural substrates. The bone-contacting side is treated to impart improved osteoinductive/osteoconductive properties. In one example, the bone-contacting side is provided with a nano-scale textured surface promoting bone in- and on-growth. In another example, osteoinductive and osteoconductive materials may also be incorporated into or on the surface of the bone-contacting portion. As shown in this piece of prior art, the implant is shaped as a fairly thin plate with a slight convexity or concavity to follow the contour of an articulate surface in a joint.

Patent document US6306925B1 also shows an implant with a composite material comprising layers of bioactive glass reinforced with ductile layers of metal.

Patent document WO2006/08360 describes the production of a functionally graded material (FGM) net shaped body with FAST/SPS where the different materials included are a metal or a metal alloy in combination with a ceramic such as an oxide, nitride or carbide, or another metal or metal alloy.

The closet prior at is US 2004/0243241 A1, which defines the preamble of claim 1.

### Object of the Invention

The overall object of the present invention is to provide a solution to the problem of providing a small joint implant for the replacement of damaged cartilage, which can be inserted through arthroscopy, a small open surgery operation or a combination thereof. A specific object of the present invention is to provide an implant that is devised for, on one hand, an efficient long term fixation to the bone and, on the other hand, for providing a wear resistant surface to substitute for damaged cartilage.

### Problem to be solved

The overall problem to be solved by the present invention is to provide an implant with improved mechanical properties, the implant being a thin plate-shaped implant of the kind having a wear resistant articulate surface and a bioactive surface and being devised for repair of damaged cartilage.

### Aspects of the problem

The present invention further addresses the following problem aspects:
- To enable adherence between the bioactive surface and the wear resistant articulate surface.
- To improve the mechanical properties and durability of the bioactive surface.
- To improve the fixation of the implant to the bone with regard to the impact of the implant on the bone tissue.
- To provide a manufacturing method for producing an implant material and an implant.

### Summary of the invention

The present innovation relates to a new medical implant structure suitable for articular surface implants such as knee implants, where said implant can be inserted through a small surgical operation such as arthroscopy, a small open surgery or a combination thereof. The implant is provided with dual functionalities by having a first surface which is wear resistant and devised for facing the articulating part of the joint, and a second surface which is bioactive and devised for facing the bone structure underlying the cartilage. The wear resistant surface comprises a biocompatible wear resistant first metal, metal alloy or ceramic and provides a load bearing surface which is strong and hard enough to resist the wearing forces acting upon it through the movement of the joint. The bioactive surface comprises a sintered material having a homogenous microstructure comprising a mixture of a second metal, metal alloy or ceramic and a bioactive ceramic material or bioactive glass. The sintered material provides firm long-term attachment of the implant to the bone, by stimulating bone growth and bone integration, thus forming a bioactive surface, and at the same time provides a durable bone-contacting surface to the implant. The bioactive ceramic or bioactive glass promotes firm attachment to the bone while the integrated metal, metal alloy or ceramic gives the desired mechanical properties. Preferably the bioactive surface comprises a sintered material having a homogenous microstructure of stainless steel and hydroxyapatite (HA).

A consequence of the invention is that a minimal surgical operation and minimal modifications on the underlying bone and surrounding tissue are required when preparing for the implant surgery and with minimal effects on the tissue after implantation. The bioactive implant in accordance with the invention enables implantation without a rigorous initial (primary) fixation, yet yielding a durable long-lasting (secondary) fixation. This, on one hand gives a long life length of the implant in situ and on the other hand has a positive effect on the possibility for performing further surgery, in cases when such is needed, for example total joint replacement.

The present invention is defined in claim 1.

Further varieties of the inventive concept comprise such an implant comprising any of the following optional individual or combinable aspects:

The second surface comprises bioactive ceramic or bioactive glass in the range of 20-80 weight %.

The sintered material is achieved by electric pulse assisted consolidation (EPAC, SPS).

The sintered material structure adhering said first surface and said second surface is in the form of a functionally graded material providing a gradual change of mechanical, compositional and/or microstructural properties with position.

The functionally graded material comprises a binding structure that comprise mental material elements in common with the first metal, metal alloy or ceramic element of the first surface and with the second metal material elements in common with the second metal, metal alloy or ceramic of the second surface.

The first and/or the second metal, metal alloy or ceramic is any of stainless steel, cobalt-based alloys, chrome-based alloys, titanium-based alloys, pure titanium, zirconium-based alloys, tantalum, niobium, precious metals and their alloys, aluminium oxide, silicon nitride or yttria-stabilized zirconia.

The first metal, metal alloy or ceramic is stainless steel.

The second metal, metal alloy or ceramic is a cobalt chromium alloy.

The first metal, metal alloy or ceramic is the same as the second metal, metal alloy or ceramic.

The first metal, metal alloy or ceramic is different from the second metal, metal alloy or ceramic.

The bioactive ceramic material is chosen from the group of hydroxyapatite, calcium sulphate, calcium phosphate, calcium aluminates, calcium silicates, calcium carbonates or combinations thereof, or bioactive glass.

The bioactive ceramic material is hydroxyapatite.

The first surface comprises stainless steel, and;
the second surface comprises a sintered material having a homogenous microstructure comprising a mixture of stainless steel or a cobalt chromium alloy and a bioactive ceramic material or bioactive glass.

The first surface comprises stainless steel sintered from stainless steel raw material particles having a particle size which is less than 25 µm;
the second surface comprises a sintered material having a homogenous microstructure comprising a mixture of stainless steel or a cobalt chromium alloy and a bioactive ceramic material or bioactive glass;
the adherence between the first and the second surfaces is achieved by a porous layer configured between the first and the second surfaces comprising stainless steel sintered from stainless steel raw material particles having a particle size which is larger than 75 µm.

The adherence between the first and the second surfaces is achieved by a sintered material structure comprising
one or more layers configured between the first and the second surfaces, each layer comprising a sintered mixture having a homogenous microstructure of the biocompatible metal, metal alloy or ceramic and the bioactive ceramic or bioactive glass, wherein
the ratio of the bioactive ceramic material to stainless steel gradually changes over the layers, such that the layers comprise an increasing ratio of the bioactive ceramic material when going from the first surface towards the second surface layer.

The material structure of the second surface is adapted further to stimulate bioactivity, for example by being porous and/or rough.

The medical implant further comprising primary fixation means (4) for mechanical attachment to the bone, for example a screw, peg, keel or barb.

The primary fixation means (4) comprises the bioactive ceramic or bioactive glass of the second surface.

The primary fixation means (4) comprises the biocompatible metal, metal alloy or ceramic of the first surface.

The primary fixation means (4) is formed as a protrusion from a first surface layer (10) extending through a second surface layer (11) and possible intermediate layer or layers (12).

The implant is designed as a thin plate or plate-like structure, possibly concave/convex, where the area of the first surface is between 0.5 cm² and 15 cm², preferably between 1 cm² and 10 cm², and the thickness between the first and the second surfaces is between 1 mm and 10 mm.

The first surface (5) or the second surface (7) is surface treated, for example by polishing, heat treatment, precipitation hardening or deposition of a suitable surface coating.

Other aspects and features of the inventive concept are described in the detailed description below.

### Brief description of the figures

The invention will be explained in more detail in the following description, referring to the enclosed figures, where:
Fig 1A-C show medical implants according to different embodiments of the present invention;
Fig 2 shows a first alternative of a functionally graded material of a medical implant according to an embodiment of the present invention;
Fig 3 shows a second alternative of a functionally graded material of a medical implant according to an embodiment of the present invention;
Fig 4 shows a third alternative of a functionally graded material of a medical implant according to an embodiment of the present invention;
Fig 5A-B show medical implants according to different embodiments of the present invention, inserted into the bone of a joint to replace damaged cartilage;
Fig 6A-C show medical implants according to different embodiments of the present invention;
Fig 7 shows the microstructure of the second surface of the medical implant according to an embodiment of the present invention.

### Detailed description of the invention

### Introduction

The present innovation relates to a new medical implant structure and material and use of such a material in a medical implant for an articular surface implant, as well as a method for manufacturing such a medical implant and medical implant structure. The implant is suitable for replacement of cartilage in the knee joint, to stop or retard further cartilage break-down. The invention may however have other useful applications, such as in a medical implant for an articulating surface of any other joint in the body, e.g. elbow, ankle, finger, hip, toe and shoulder.

### Implant structure

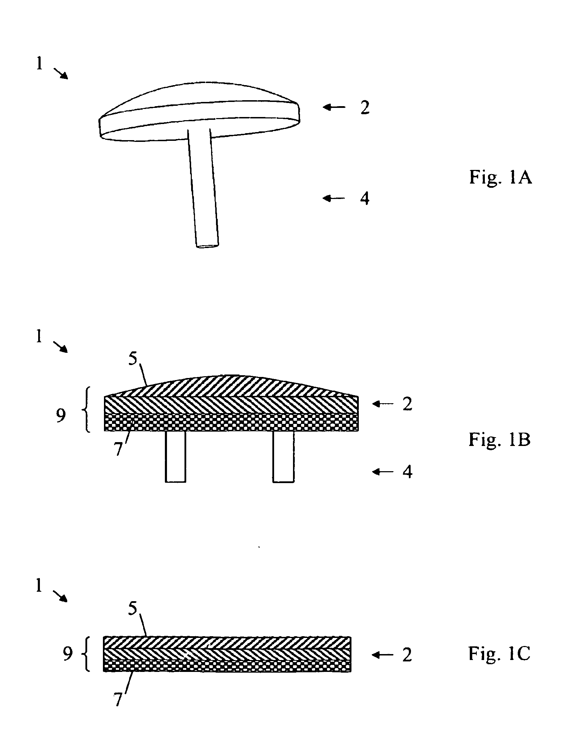

Fig 1A-C show schematic views of different embodiments of a medical implant in accordance with the invention. Fig 1A shows the medical implant at an angular view from above. Fig 1B and C show the medical implant from a side view. The medical implant 1 comprises an implant body 2 and, in the examples shown in Fig 1A and B, a primary fixation means 4 extending from the implant body 2. The purpose of the primary fixation means 4 is primarily to provide mechanical attachment of the implant to the bone in immediate connection with the surgical operation. The implant body 2 has a thin, plate-like design. The plate can vary in size and shape and may be adjusted to the size and shape of the damaged cartilage tissue and to the needs of particular treatment situations. For instance the cross-section of the implant body 1 may have a circular or roughly circular, oval, triangular, square or irregular shape. The size of the implant 1 may also vary. The surface area of the implant body 2 varies. between 0,5 cm² and 10 cm² or preferably between about 1 cm² and 10 cm². In general, small implants are preferred since they have a smaller impact on the joint at the site of incision and are also more easily implanted using arthroscopy or smaller open surgical procedures. The primary factor for determining the size of the implant is however the nature of the lesion to be repaired. The thickness of the implant body 2 is between 1 mm and about 10 mm, preferably between about 2 mm and 5 mm. The thickness of the implant should on the whole preferably match the thickness of the original cartilage layer, possibly also adapted to adjust for the recess in the bone, used for anchorage of the implant (see further explanation below) or formed as a part of the disease process.

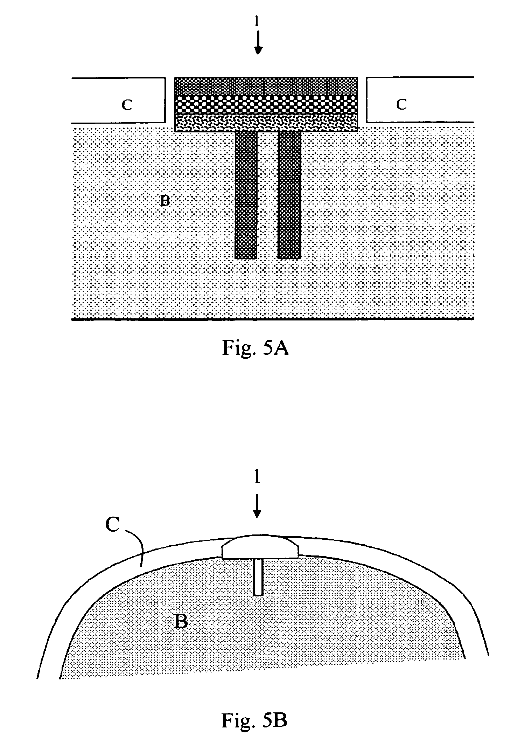

The body 2 of the medical implant 1 comprises a first surface 5, which is configured to face the articulating part of the joint and is wear resistant, and a second surface 7, which is configured to face the bone and is bioactive. The first surface 5 should preferably have a profile which on the whole matches the curvature of the original anatomical surface at the site of incision, as e.g. illustrated in Fig 5B. It is e.g. typically convex for implants intended for the femoral side of a knee joint, but can also have multiple curvatures, In another embodiment adapted for concave articulate surfaces, the first surface of the implant body has a corresponding concave shape. The second surface 7, i.e. the surface facing the bone, should typically be flat or concave or may be convex to fit the underlying bony surface where the cartilage in the implant site is removed and the bone possibly is prepared with a recess for the implant.

### Wear resistant surface - First surface

The first, wear resistant, surface 5, which is also the articulate surface of the medical implant, comprises a biocompatible metal, metal alloy or ceramic, preferably stainless steel. The metal, metal alloy or ceramic is chosen to provide a wear resistant surface which is durable and resistant to the abrasive forces acting upon it as it articulates and moves in relation to the surrounding parts of the joint. The wear-resistant biocompatible material may consist of a metal, a metal alloy or a ceramic material. More specifically it can consist of any metal or metal alloy used for structural applications in the body, such as stainless steel, cobalt-based alloys, chrome-based alloys, titanium-based alloys, pure titanium, zirconium-based alloys, tantalum, niobium and precious metals and their alloys. If a ceramic is used as the biocompatible material, it can be a biocompatible ceramic such as aluminium oxide, silicon nitride or yttria-stabilized zirconia.

Stainless steel is a material which is well documented for the application in implants and prostheses. It also provides a material which is hard and strong enough to withstand and tolerate the large mechanical forces and heavy and changing work loads subjected to it in the joint. Different grades exist where the properties have been optimised for applications in the human body. An example of a stainless steel for use in an embodiment of the present invention is an alloy mainly comprising iron, carbon, chromium (12-20%), molybdenum (0.2-3%), and nickel (8-15%).

It should also be understood that the first, wear resistant, surface 5 may also be further surface treated in order to e.g. achieve an even more durable surface or a surface with a lower friction coefficient. Such treatments may include, for example, polishing, heat treatment, precipitation hardening or depositing a suitable surface coating.

### Bioactive surface - Second surface

The second, bioactive, surface 7 of the medical implant 1, which is also the bone-contacting surface, comprises a sintered material having a homogenous microstructure of metal, metal alloy or ceramic and a bioactive ceramic material or bioactive glass. The purpose of the bioactive surface 7 is to provide long-term firm adherence of the medical implant 1 to the underlying bone, by stimulating the bone to grow into or onto the implant surface. Several bioactive materials that have a stimulating effect on bone growth are known and have been used to promote adherence between implants and bone. Examples of such prior art bioactive materials include bioactive glass, bioactive ceramics and biomolecules such as collagens, fibronectin, osteonectin and various growth factors. A commonly used bioactive material in the field of implant technology is the bioactive ceramic hydroxyapatite (HA). HA is the major mineral constituent of bone and is able to slowly bond with bone *in vivo.* Thus, HA coatings have been developed for medical implants to promote bone attachment. Unfortunately HA is a brittle material and the bonding between HA and prior art metallic implants is weak and subject to fracture. Another bioactive material commonly used in prior art is bioactive glass. Bioactive glasses, generally comprising SiO₂, CaSiO₃, P₂O₅, Na₂O and/or CaO and possibly other metal oxides or fluorides, are able to stimulate bone growth faster than HA, but are also weak and in themselves lack the necessary mechanical properties required by articulate joint implants.

Previous attempted solutions for providing a bioactive surface on a medical implant have been e.g. to deposit or spray bioactive material onto the surface of an implant, to adsorb bioactive material to the surface or to design a layered composite material of e.g. metal and bioactive glass. As indicated above, these solutions suffer from the disadvantage that bioactive material which is deposited, sprayed or adsorbed onto the surface of the implant is prone to wear or peel off. For example, both HA and bioactive glass are brittle and have not this far been enabled to adhere firmly to the body of the implant. Also, the layered solution, where bioactive glass is reinforced by metallic layers or thin metal foils, is prone to cracking and peel.

In the present invention, a bioactive surface 7 is provided by incorporating by sintering a bioactive ceramic or bioactive glass into a metal, metal alloy or ceramic. Thereby, a sintered mixture material having a more or less homogenous microstructure comprising the bioactive ceramic or bioactive glass and the metal, metal alloy or ceramic is formed. In this way the bioactive ceramic or glass is fully integrated with and supported by the metal, metal alloy or ceramic structure. The bioactive ceramic or bioactive glass promotes firm attachment to the bone while the metal, metal alloy or ceramic gives the desired mechanical properties. Thus the second surface 7 is a bioactive surface which is strong enough to be integrated with the bone without breaking, which is resistant to wearing and peel and at the same time adheres firmly to the body 2 of the medical implant 1.

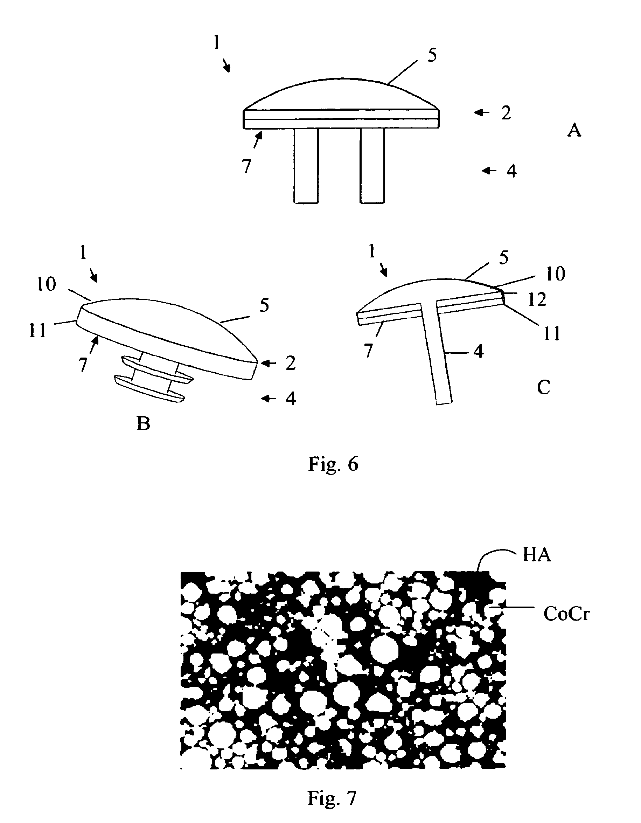

In the homogenous microstructure the constituent material components are evenly distributed in the structure. It should be understood that a complete homogeneity is desired but in practise not readily achievable, thus the microstructure may consequently comprise less homogeneous portions. Fig 7 shows an example of a microstructure in an embodiment of the present invention. The figure shows the microstructure of a sintered composition of a cobalt chromium alloy CoCr and hydroxyapatite HA (50:50). The white matter is CoCr and the black is HA. HA forms a continuous phase while the CoCr forms scattered particles.

Generally, the content of the bioactive ceramic or bioactive glass in the second surface is more than 20 % and preferably more than 30 %. This high ratio of the bioactive material combined with satisfying mechanical properties is enabled by the sintering of the bioactive material with a metal, metal alloy or ceramic according to the invention.

The bioactive ceramic of the sintered mixture material of the second surface 7 is preferably hydroxyapatite (also called hydroxylapatite, HA). Hydroxyapatite has the chemical composition Ca₅(PO₄)₃(OH), but is usually written Ca₁₀(PO₄)₆(OH)₂ to denote that the crystal unit cell comprises two molecules. The bioactive material may also be any of the ceramics calcium sulphate, calcium phosphate, calcium aluminates, calcium silicates, calcium carbonates or bioactive glass, or combinations thereof.

The metal, metal alloy or ceramic content of the bioactive, second surface 7 is selected from any metal, metal alloy or ceramic used for structural applications in the body, such as stainless steel, cobalt-based alloys, chrome-based alloys, titanium-based alloys, pure titanium, zirconium-based alloys, tantalum, niobium, precious metals and their alloys, as well as aluminium oxide, silicon nitride or yttria-stabilized zirconia.

The metal, metal alloy or ceramic of the sintered mixture material of the second surface 7 is preferably a cobalt chromium alloy CoCr or stainless steel, or another suitable metal, metal alloy or ceramic. In a preferred embodiment the second surface comprises a cobalt chromium alloy CoCr or stainless steel and HA in a ratio in the range of 20:80 to about 80:20, for example 70:30, 50:50 or 30:70.

Further, small amounts, up to 10 wt%, of sintering additives such as oxides of phosphorous, sodium, magnesium, potassium, silver, aluminium, titanium and/or silicon can be added to the hydroxyapatite powder to facilitate the sintering process.

The second surface, i.e. the surface of the bioactive material can be rough or porous in order to facilitate the attachment of the implant to the bone.

### Adherence between surfaces

The first, wear resistant, surface 5 and the second, bioactive, surface 7 are fixed or adhered to one another by means of a sintered material structure that in different embodiments is realized in different manners. In one variety a first surface layer comprising the first wear resistant surface 5 is sintered to a second surface layer comprising the second bioactive surface 7. In another variety there is provided an intermediate layer sintered between the first surface layer and the second surface layer.

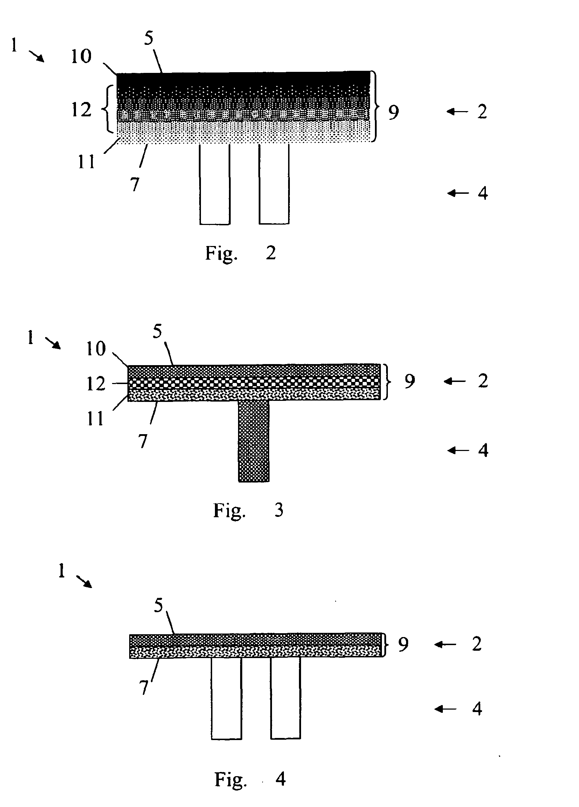

Fig 2 and Fig 3 show two different embodiments where the surfaces are adhered by different variants of functionally graded materials 9. A functionally graded material (FGM) is generally characterised by a gradual change of material properties with position. It is an anisotropic composite material where a gradient deliberately has been introduced into the material. This gradient can for example be in composition and/or microstructure and/or mechanical properties. The gradual change with position provides for firm adhesion between the first and the second surfaces, or, expressed differently, between layers of material occurring between the surfaces. Furthermore, the gradual change of material properties yields a gradual transition in mechanical, structural and/or chemical properties, which leads to higher durability of the material. It should be understood that the term functionally graded material means that there is some kind of compatibility between different layers or surfaces of the implant body structure. The gradual change may be realized as a linear or continuous change in properties and/or discrete changes between two or more distinct, more or less well defined layers that have some property in common to make them mutually compatible.

An example of an FGM 9 in an embodiment of the present invention, shown in Fig 2 and exemplified in more detail below, is a gradual change in composition from a pure or substantially pure metal, metal alloy or ceramic in the first surface to a gradual increase in the concentration/ratio of bioactive ceramic or bioactive glass in a sintered mixture material, towards the second surface. The gradual change is achieved either as a linear change or as a gradual change between two or more distinct, well defined layers. The FGM 9 in the embodiment exemplified in Fig 2 comprises six distinct layers that are adhered and compacted by sintering; one first surface layer 10 of the first surface 5 which comprises a wear resistant metal, metal alloy or ceramic, one second surface layer 11 of the second surface 7 which comprises a sintered mixture material comprising a metal, metal alloy or ceramic and a relatively high ratio of bioactive ceramic or bioactive glass, and four intermediate layers 12 which have an increasing ratio of the bioactive material going from the first surface layer 10 towards the second surface layer 11.

In another embodiment of the present invention, schematically illustrated in Fig 3, the first 5 and the second 7 surfaces are adhered by an FGM 9 which provides a gradual change in properties but not necessarily in composition. The FGM of this embodiment comprises one or more intermediate layers 12 which comprise a material which is distinct from the material of the first 5 and/or the second 7 surfaces. Such an intermediate layer may e.g. comprise steel, titanium, and hydroxylapatite to obtain improved compatibility between the layers and thereby reduce the number of layers and avoid undesired cracks in the layers. Such an embodiment could have for example titanium, a titanium alloy or a ceramic material as the third material between the wear-resistant biocompatible material and the bioactive material. In a preferred embodiment the first surface 5 comprises a relatively dense stainless steel, sintered using steel of a small particle size (<25 µm), the second surface 7 comprises a composite of relatively dense cobalt chromium alloy CoCr or stainless steel and bioactive ceramic or glass, and the intermediate layer 12 comprises relatively porous stainless steel, sintered using steel of a large particle size (>75 µm). The porous intermediate layer 12 picks up and distributes the tension and forces that build up between the first 5 and the second 7 surfaces and thus prevents the surfaces from cracking, thereby providing a durable material.

In the embodiments illustrated in Fig 2 and Fig 3 one or more intermediate layers 12 form a sintered bonding structure that provides adherence between the first, wear resistant and biocompatible surface 5 and the second, bioactive surface 7 as well as mechanically stabilizing properties to the implant structure. The functionally graded material is in one embodiment as shown in Fig 4 and Fig 6C realized with two layers, i.e. a first surface layer 10 comprising the first, wear resistant and biocompatible surface 5 sintered together with a second surface layer 11 comprising the second, bioactive surface 7.

### Primary fixation

The long-term integration, herein called secondary fixation, of the implant with the bone, stimulated by the bioactive ceramic or bioactive glass, evolves over time. In order to promote more immediate attachment of the implant to the bone as it is implanted into the body, the implant may also be designed with a small device 4 for immediate, mechanical attachment, herein called primary fixation. The primary fixation means 4 may e.g. be devised as a physical structure, glue, bone cement or the like. By physical structure is meant a protrusion, such as one or more of a small screw, peg, keel, barb or the like, as exemplified in e.g. Fig 1A and 1B. Alternatively, as shown in Fig 1C, the medical implant 1 may lack such primary fixation means or e.g. be devised with a bore through which a bone screw or the like can be inserted and fastened into the bone. Other possible alternatives are illustrated in Fig 6A-C, showing different embodiments of the primary fixation means 4, as well as medical implants 1.

The primary fixation means 4 may, if configured as a physical structure, comprise e.g. the metal, metal alloy or ceramic, as in the first surface, or a sintered mixture material of a metal, metal alloy or ceramic and a bioactive ceramic or bioactive glass, as in the second surface. Fig 6C shows an embodiment where the primary fixation means 4 is formed as a protrusion extending from the first surface layer 10 through the second surface layer 11 and possible intermediate layer or layers 12.

These primary fixation means, e.g. protrusions in the shape of pegs, comprise in one embodiment the bioactive material and have a length of 1 to 10 mm, typically 2-5 mm, and a diameter of 1 to 5 mm, typically 2 to 4 mm. In another embodiment, the length is between 1 and 20, and typically 3-10 mm, and the diameter is typically 1-4 mm. When in place in the joint, the protrusions should penetrate the sub-chondral bone plate and engage the cancellous bone. The protrusion will give stability and promote the attachment of the bioactive material to the bone.

### Method for manufacture

The implant and FGMs comprised in the implant can be prepared through different techniques such as conventional powder metallurgy processing, vapour deposition and sintering techniques. The preferred technique for producing said implant is consolidation through sintering. The sintering technique is preferably electric pulse assisted consolidation (EPAC), also referred to as spark plasma sintering (SPS), pulsed electric current sintering (PECS), field assisted sintering technique (FAST), plasma-assisted sintering (PAS) and plasma pressure compaction (P²C). Electric pulse assisted consolidation includes processes based on heating a material to be compacted with a pulsed DC current. The process allows very rapid heating under high pressures. This process, hereafter referred to as SPS, has proved to be very well suited for the production of functionally graded material. SPS gives advantages such as no need of binders in the powders and a controlled shrinkage of the material during the compaction. Further, the possibility to rapidly change the temperature and pressure makes it easier to tailor the microstructure of the material and to optimize the sintering conditions compared to conventional compaction techniques.

The general way to form a FGM through spark plasma sintering today is to build it up layer by layer with different compositions. Further, prior to sintering, the powders can be pressed together and thereby form a so called green body, which is an un-sintered item to become a ceramic upon sintering, which is later on inserted into the SPS unit to be sintered.

The spark plasma sintering technique is used for manufacturing the gradient components, i.e. the surfaces and the layers, in an exemplifying embodiment of this invention. However, gradient materials produced through alternative methods can also be used for the same purpose, for example sintering methods such as hot pressing, hot isostatic pressing or pressureless sintering. SPS combines rapid heating, a short holding time at the desired sintering temperature and a high pressure for sintering of the components.

The pressure used during the process of the present invention is between 10 and 150 MPa, preferably between 30 and 100 MPa. The heating rate applied is between 5 and 600 °C min⁻¹, preferably 50-150 °C min⁻¹. The sintering temperature and time are chosen so that a total or near total densification, implying a density of at least 95%, or at least 97% of the theoretical density, will be obtained for the layer comprising essentially 100 wt% biocompatible wear resistant metal, metal alloy or ceramic. This temperature is between 800°C and 1300°C, typically 900°C -1 100°C. The holding time during the sintering process is between about I minute and about 30 minutes, typically between about 2 minutes and about 10 minutes.

The SPS technique uses a combination of a high current and a low voltage. A pulsed DC current with typical pulse durations of a few ms and currents of 0.5-30 kA flows through the punches, die and, depending on the electrical properties of the specimen, also through the specimen. The electrical pulses are generated in the form of pulse packages where the on: off relation is in the region of 1:99 to 99:1, typically 12:2 (12 pulses on, 2 off). The pressure is applied on the two electrically conducting punches of the powder chamber, in a uniaxial direction.

The sintering procedure preferably also includes a high heating rate (a steep heating ramp) and a short holding time at the desired sintering temperature. The cooling down of the sample can either be programmed or the sample will cool down automatically as the current is switched off.

### One-step sintering production method

In a general overview of a single sintering step production method, the method comprises the following steps.

The component is produced through sintering in a single step, said production process comprising the steps:
i) Placing powder or a green body in a die, said powder or green body consisting of at least two kinds of raw material. The powder placed in the die comprises the materials for both the wear resistant surface, the bioactive surface and, if present, a protrusion forming a primary fixation means.
ii) Consolidating the material of step i) by using a sintering technique.
iii) Possibly machining the sintered body so a protrusion is formed;

The fully sintered component may after the sintering process be treated with different methods to obtain a finished product, said methods may comprise surface machining, blasting, etching, grinding and polishing.

A more detailed example of a single step individual fitting production method adapted for tailor made production of an embodiment of the implant comprises the following steps:
(i) inserting the powders of different compositions stepwise, or a pre-formed green body, into a conductive graphite die (chamber);
(ii) closing the graphite die chamber with two electrically conducting punches and inserting the die into the SPS unit;
(iii) applying a uniaxial pressure on the two punches of the graphite die, thereby applying a pressure on the material
(iv) heating the material with a pulsed electrical energy, going through the punches and the electrically conducting die, during a desired time until the sintering is completed.

### Multistep sintering production method

In a general overview of a multistep production method, the method comprises the following steps.

In general, the component is produced through sintering in multiple steps, said production process comprising the steps:
i) Placing powder or a green body in a die, said powder or green body consisting of at least one kind of raw material.
ii) Consolidating the material of step i) by using a sintering technique.
iii) Machining the sintered body so that a protrusion for a primary fixation means is formed.
iv) Placing more material or another green body onto the machined body.
v) Consolidating the material of step iv) by using a sintering technique.

In the multi step mehtod mentioned above, step i) comprises the materials for the wear resistant side and the protrusion, while step iv) comprises the material for the bioactive surface.

In another method, step i) above comprises at least two different kinds of materials, resulting in different materials on the wear resistant surface and in the protrusion forming primary fixation means.

The fully sintered component may after the sintering process be treated with different methods to obtain a finished product, said methods may comprise surface machining, blasting, etching, grinding and polishing.

### Different embodiments and examples of the present invention

A functionally graded material (FGM) is characterised by a gradual change of material properties with position. An FGM 9 may in some embodiments, as the one schematically shown in Fig 3, be achieved by one or more intermediate layers 12, which provide a gradual change in properties, but not necessarily in composition, within the FGM structure. The FGM 9 of the exemplifying embodiment comprises one or more intermediate layers 12 which comprise a material which is distinct from the material of the first surface 5 and the corresponding first surface layer 10 and/or the second surface 7 and the corresponding second surface layer 11. The intermediate layer(s) 12, although having a different material composition, provides mechanical and/or structural properties that are intermediate to the properties of the first and the second surface layers 10, 11. Additionally or alternatively it provides mechanical properties which can distribute or pick up forces and tension that would build up between the first and the second surface layers 10, 11 and therefore make them more prone to cracking.

In a preferred embodiment the FGM has a first surface layer 10 comprising the first surface 5, comprising relatively dense stainless steel, having a relative density of at least 95-97%, sintered using steel powder having a small particle size (<25 µm). The second surface layer 11 comprising the second surface 7, comprises a sintered mixture material with a cobalt chromium alloy CoCr or stainless steel and a bioactive ceramic or bioactive glass, sintered e.g. using a cobalt chromium alloy CoCr or stainless steel powder having a particle size of <50 µm, or preferably about <22 µm. The intermediate layer 12 comprises a layer of relatively porous stainless steel, with a relative density between 50 and 98%, preferably between 60 and 95%, sintered using steel powder having a large particle size (>75 µm). The porous intermediate layer 12 picks up and distributes the tension and forces that build up between the first 5 and the second 7 surfaces and thus prevents the surfaces from cracking. In various forms of this embodiment the metal, metal alloy or ceramic of the different layers may be of varying composition, e.g. having a composition of <0.3 % carbon, 2-3 % molybdenum, <0.0455% phosphorous, <1% silicon, 10-14% nickel, 16-18% chromium, <2% manganese, <0.03% sulphur and iron to balance for the first wear resistant surface, and a cobalt-chromium alloy without nickel in the surfaces facing the bone. Such an alloy can for example have the composition 0.2-0.3 % carbon, 5-7 % molybdenum, 0.15-0.2 % nitrogen, 26-30% chromium and cobalt The ratios (%) are herein indicated in percentage per weight. An advantage of the FGM of the described embodiment is that it may be manufactured so as to give a medical implant having an implant body 2 which is very thin.

The embodiment described above may comprise primary fixation means 4, as for example illustrated in Fig 3. The primary fixation means 4 may be of various physical forms, as described above. It may comprise the wear resistant first metal, metal alloy or ceramic of the first surface 5 or the sintered mixture material of the second surface, having a second metal, metal alloy or ceramic and a bioactive ceramic material/bioactive glass, or another suitable material. In a preferred embodiment, having manufacturing advantages, the medical implant 1 comprises a first surface 5 of stainless steel, a second surface 7 of a sintered mixture material of stainless steel and hydroxyapatite and primary fixation means 4 of stainless steel. In a preferred variant of said embodiment the medical implant also comprises an intermediate layer 12 of porous stainless steel, sintered using steel powder having a large particle size (>75 µm).

A method for producing an implant with a biocompatible wear resistant material having a transition to a bioactive part, said biocompatible wear resistant material being a metal or metal alloy, comprises the steps:
i) Forming a powder body, comprising at least one material, comprising the material of the wear resistant surface and the protrusion.
ii) Consolidating the material of step i) by using a sintering technique; wherein the wear resistant surface has a relative density of at least 95 % of the theoretical density.
iii) Machining the sintered component so that a protrusion for a primary fixation means is formed.
iv) Placing morse material onto the machined body, said material being selected for the purpose of creating adherence between the two surfaces and said material comprising a metal or metal alloy of a coarser grain size than the material of the wear surface.
v) Placing the material of the bioactive surface on top of the adherence material, said bioactive surface material comprising a mixture of a metal or metal alloy and a bioactive ceramic or bioactive glass material wherein the ratio between said wear resistant metal or metal alloy and said bioactive material is in the range of 20:80 to about 80:20, or in the range of 30:70 to about 70:30.
v) Consolidating the material of step iv) by using a sintering technique
vi) Possibly machining the double sintered body to a desired curvature of the surfaces

An FGM may be achieved by a gradual change of material composition with position. In one embodiment of the invention, the medical implant comprises an FGM 9 designed with two or more layers comprising a gradual change in the ratio of wear resistant metal, metal alloy or ceramic to bioactive ceramic or glass. The implant body 2 in the embodiment exemplified in Fig 2 comprises an FGM 9 having several layers that gradually changes in the ratio of the wear resistant material to the bioactive material. For example, the outermost layer and surface, which is to face the articulate part of the joint, may consist entirely, or almost entirely of the wear resistant material. The layer adjacent to said outermost layer then comprises a small ratio of the bioactive material, the next layer a larger ratio of the bioactive material and so forth. The layer and surface which is to face the bone then comprises the largest ratio of the bioactive material, compared to the other layers. For example, the ratio between said wear resistant material to said bioactive material may vary in the range, going from the first, wear resistant surface towards the second, bioactive surface, of 100:0 to about 0:100, or in the range of 100:0 to about 30:70, or in the range of 100:0 to about 50:50.

The gradient material of this embodiment of the invention consists of a number of layers, typically between 4 and 25, or more typically between 7 and 20, with different compositions in each layer. Preferred embodiments comprise between 3 and 25, or more typically between 3 and 10 layers. The outermost layers of the component consist of the biocompatible material, forming a load-bearing surface, and the bioactive material, with or without addition of the wear-resistant material for improved stability, forming a bone-contacting surface, respectively. A more detailed description of the structure of this embodiment is given below in the section describing a method for producing the embodied structure.

The present method also provides a functionary graded material, where one of the layers essentially comprises stainless steel and one of the layers essentially comprises the bioactive material, and other layers, if present, essentially comprise a mixture of stainless steel and the bioactive material.

Another preferred method also provides a functionally graded material, where the first surface 5 essentially comprises dense stainless steel and the second surface 7 comprises a sintered mixture material of a cobalt chromium alloy CoCr or stainless steel and a bioactive ceramic/glass material, and other layers, if present, comprise a mixture of stainless steel and the bioactive ceramic or glass material, ranging in ratio of stainless steel:bioactive ceramic material between 0:100 and 70:30. Preferably the bioactive ceramic material is hydroxyapatite.

Another method for producing a multi-layer design FGM with a biocompatible wear resistant material having a gradual transition to a bioactive part, said biocompatible wear resistant material being a metal or metal alloy comprises the steps:
i) forming a gradient materials composed of at least 4 layers, wherein one layer essentially comprises 100 wt% biocompatible wear resistant metal or metal alloy, and the other layers each essentially comprise a powder of a biocompatible wear resistant metal or metal alloy, together with a powder of a bioactive material, or the bioactive material only, wherein each of the layers differ in the ratio between wear resistant metal or metal alloy and bioactive material;
ii) consolidating the gradient material of step i) by using a sintering technique; wherein the layer with essentially 100 wt% metal or metal alloy has a relative density of at least 95 % of the theoretical density.

In varieties of this method, the number of layers in the functionally graded material is between 4 and 25, or preferably between 5 and 15, wherein said bioactive material, or the layers comprising said bioactive material, further comprises a maximum of about 50 wt% stainless steel, or a maximum of about 30% stainless steel.

The present invention comprises a functionally graded material obtained by the above method, where one of the layers essentially comprises stainless steel and one of the layers essentially comprises the bioactive material, and other layers, if present, essentially comprise a mixture of stainless steel and the bioactive material.

### Examples

### Example 1

A gradient material of hydroxylapatite (HAP) and stainless steel (SS) was prepared. 11 different powder mixtures were prepared with the following compositions:

| Layer | wt% SS | wt% HAP |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |
| 7 | 40 | 60 |
| 8 | 30 | 70 |
| 9 | 20 | 80 |
| 10 | 10 | 90 |
| 11 | 0 | 100 |

The 11 different powders were produced through mixing stainless steel 316L (D₉₀<22 µm) and/or hydroxylapatite (D₅₀<5 µm) in a liquid medium in a ball mill for 2 h followed by drying in a conventional oven. The powders were inserted layer by layer in a graphite die chamber and the chamber was closed by two punches. The sample was sintered in a SPS unit and the temperature was initially automatically raised to 600°C. Subsequently, a heating rate of 100°C min⁻¹ was applied. The sample was densified at 1000°C for 5 minutes. The temperature was measured with an optical pyrometer focused on the surfaces of the sintering die. The sintering took place under vacuum. The pressure was kept at 100 MPa. The component was shaped as a cylinder with a diameter of 20 mm and a height of 5 mm. The layers were free of cracks.

### Example 2

A gradient material of hydroxylapatite (HAP) and stainless steel (SS) was prepared. 8 different powder mixtures were prepared with the following compositions:

| Layer | wt% SS | wt% HAP |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |
| 7 | 40 | 60 |
| 8 | 30 | 70 |

The eight different powders were produced through mixing stainless steel 316L (D₉₀<22 µm) and/or hydroxylapatite (D₅₀<5 µm) in a liquid medium in a ball mill for 2 h followed by drying in a conventional oven. The powders were inserted layer by layer in a graphite die chamber and the chamber was closed by two punches. The sample was sintered in a SPS unit and the temperature was initially automatically raised to 600°C. Subsequently, a heating rate of 100°C min⁻¹ was applied. The sample was densified at 1000°C for 5 minutes. The temperature was measured with an optical pyrometer focused on the surfaces of the sintering die. The sintering took place under vacuum. The pressure was kept at 75 MPa. The component was shaped as a cylinder with a diameter of 20 mm and a height of 4 mm. The layers were free of cracks.

### Example 3

A gradient material of hydroxylapatite (HAP) and stainless steel (SS) was prepared. 6 different powder mixtures were prepared with the following compositions:

| Layer | wt% SS | wt% HAP |
|---|---|---|
| 1 | 100 | 0 |
| 2 | 90 | 10 |
| 3 | 80 | 20 |
| 4 | 70 | 30 |
| 5 | 60 | 40 |
| 6 | 50 | 50 |

The six different powders were produced through mixing stainless steel 316L (D₉₀<22 µm) and/or hydroxylapatite (D₅₀<5 µm) in a liquid medium in a ball mill for 2 h followed by drying in a conventional oven. The powders were inserted layer by layer in a graphite die and the die was closed by two punches. The sample was sintered in a SPS unit and the temperature was initially automatically raised to 600°C. Subsequently, a heating rate of 100°C min⁻¹ was applied. The sample was densified at 950°C for 5 minutes. The temperature was measured with an optical pyrometer focused on the surfaces of the sintering die. The sintering took place under vacuum. The pressure was kept at 100 MPa. The component was shaped as a cylinder with a diameter of 20 mm and a height of 3 mm. The layers were free of cracks.

### Example 4

A knee with damaged cartilage was scanned with CT and the result thereof was converted to a CAD-drawing. Graphite tools for the SPS chamber were formed according to the requirements from the CAD-drawing in order to sinter a tailor made component in the graphite tool. The lower punch had two holes for formation of the bioactive protrusions. A stainless steel/hydroxylapatite gradient material was formed through spark plasma sintering according to Example 1, with 11 different layers. The sample was densified at 1000 °C for 5 minutes. The sintering took place under vacuum and the pressure was 75 MPa.

### Example 5 (comparative example)

A gradient material of hydroxylapatite (HAP) and stainless steel (SS) was prepared. 6 different powder mixtures were prepared with the following compositions:

| Layer | wt% SS | wt% HAP |
|---|---|---|
| 1 | 100 | 0 |
| 2 | 80 | 20 |
| 3 | 60 | 40 |
| 4 | 40 | 60 |
| 5 | 20 | 80 |
| 6 | 0 | 100 |

The six different powders were produced through mixing stainless steel 316L (D₉₀<22 µm) and/or hydroxylapatite (D₅₀<5 µm) in a liquid medium in a ball mill for I h followed by drying in a conventional oven. The powders were inserted layer by layer in a graphite die and the die was closed by two punches. The sample was sintered in a SPS unit and the temperature was initially automatically raised to 600°C. Subsequently, a heating rate of 100°C min⁻¹ was applied. The sample was densified at 1000°C for 5 minutes. The temperature was measured with an optical pyrometer focused on the surfaces of the sintering die. The sintering took place under vacuum. The pressure was kept at 75 MPa. The component was shaped as a cylinder with a diameter of 20 mm and a height of 3 mm. The pure hydroxylapatite layer at one side of the cylinder cracked and the experiment was therefore not successful. Compared to the material according to this example, the functionally graded material according to the invention is more mechanically stable.

### Example 6

An implant component consisting of two different compositions of medical stainless steel and hydroxyapatite was prepared. Further, different grain sizes of one of the stainless steel grades were applied. The component was sintered in two steps. One layer of stainless steel powder Micro-Melt 316L (d₅₀<22 µm, Carpenter Technology) was first placed into a graphite die of a diameter 15 mm. On top of that layer, Micro-melt CCM+ stainless steel powder (44-105 µm, Carpenter Technology) was placed on top of the 316L powder. The graphite die was closed by two graphite punches and the materials were sintered in a SPS unit. The temperature was initially automatically raised to 600 °C and subsequently a heating rate of 100 °C min⁻¹ was applied. The sample was densified at 950 °C for 5 minutes. The temperature was measured with an optical pyrometer focused on the surfaces of the sintering die. The sintering took place under vacuum. The pressure was kept at 75 MPa. The component was shaped as a cylinder with a diameter of 15 mm and a height of 10 mm.

The sintered cylinder was put in a turning machine and was turned so the Micro-melt CCM+ part was shaped into a protrusion.

The shaped stainless steel component was thereafter put back into a graphite die. A layer of Micro-melt CCM+ stainless steel (177-420 µm, Carpenter Technology) was put on top of the 316L material on the same surface as the protrusion. A powder was prepared through mixing 50/50 (wt%) of Micro-Melt CCM+ stainless steel (d₅₀<22 µm, Carpenter Technology) and hydroxyapatite (d50<5 µm). A layer of this powder mixture was placed on top of the coarse grained CCM+ powder. The sample was again densified, according to the set-up above with a sintering temperature of 950 °C, a pressure of 75 MPa and a sintering holding time of 5 min. Following the sintering, the wear resistant surface was shaped to obtained desired curvature. The implant component was thereafter blasted (shot peening) to remove graphite from the surface as well as to create a roughness on the bioactive surface. The wear resistant surface was polished.

### Example 7

An implant component consisting of two different compositions of medical metal alloys and hydroxyapatite was prepared. Further, different grain sizes of one of the metal alloy grades were applied. The component was sintered in two steps. One layer of stainless steel powder Micro-Melt 316L (d₅₀<22 µm, Carpenter Technology) was first placed into a graphite die of a diameter 15 mm. Micro-Melt CCM+ cobalt-chrome powder (44-105 µm, Carpenter Technology) was placed on top of the 316L powder. The graphite die was closed by two graphite punches and the materials were sintered in an SPS unit. The temperature was initially automatically raised to 600 °C and subsequently a heating rate of 100 °C min⁻¹ was applied. The sample was densified at 950 °C for 5 minutes. The temperature was measured with an optical pyrometer focused on the surfaces of the sintering die. The sintering took place under vacuum. The pressure was kept at 75 MPa. The component was shaped as a cylinder with a diameter of 15 mm and a height of 10 mm.

The sintered cylinder was put in a milling cutter and was machined so the Micro-Melt CCM+ part was shaped into a two protrusions.

The shaped metal alloy component was thereafter put back into a graphite die. A powder was prepared through mixing 50/50 (wt%) of Micro-Melt CCM+ cobalt chrome powder (d₅₀<22 µm, Carpenter Technology) and hydroxyapatite (d₅₀<5 µm). A layer of this powder mixture was placed on top of the coarse grained CCM+ powder. The sample was again densified, according to the method above with a sintering temperature of 950 °C, a pressure of 75 MPa and a sintering holding time of 5 min. Following the sintering, the wear resistant surface was shaped to obtain desired curvature. The implant component was thereafter blasted (shot peening) to remove graphite from the surface as well as to create a roughness on the bioactive surface. The wear resistant surface was carefully polished.

### Implant surgery - Positioning and placement of implant

Fig 5A and 5B show schematically embodiments of the implant I according to the present invention positioned in the cartilage C of an articulate surface of a joint. The implant 1 is levelled in a prepared recess in the cartilage C and the underlying bone B, and the primary fixation means 4 projects deeper into the bone. The thickness of the implant body and the prepared recess made into the bone are adapted such that the highest point of the implant is positioned at level with or possibly slightly above the contour surface of the cartilage. This positioning is important on one hand to avoid wearing of the surrounding cartilage C as well as wearing on the facing articulate surface. Fig 5B shows how cartilage has re-grown onto the implant 1 some time after the implant surgery.

The implant is introduced into the joint by surgical treatment, such as by means of a closed procedure, i.e. by arthroscopy as opposed to open surgery, or a small open surgical operation which is much smaller than operations for today's knee prostheses. This provides for minimal postoperative morbidity. The implant is not conceived of as a joint prosthesis but rather as an artificial biomaterial for installation into a portion of an articular surface, i.e. for cartilage replacement. Prosthetic replacement may become necessary at a later stage and the present procedure should preferably not interfere with subsequent joint arthroplasty. It is therefore important with an implant, according to the present innovation, that is not interfering with a possible future prosthesis. The use of biomaterials in this procedure implies, as has been explained above, a wear-resistant component as well as a bioactive component, as opposed to biological solutions using cell or tissue transplants.

The implant according to the present invention is, as mentioned above, suitable for the knee joint, but the implant is also useful for other joints such as elbow, ankle or finger joints. The knee joint is however the joint most often suffering from the conditions approachable by this implant.

For the surgical treatment, either of two situations may be at hand: 1) a chondral fracture, where the sub-chondral bone plate typically is completely denuded of cartilage over a confined area with sharp edges to the surrounding intact cartilage or 2) a focal area of degenerated cartilage.

Two surgical methods of the present invention may comprise the following steps:
Method 1. With a chondral fracture, the implant is preferably manufactured to be a perfect fit to the cartilaginous defect as outlined by preoperative CT/MRI investigations, or the place of the cartilaginous defect is pre-processed to match the dimensions of an implant. Ordinary arthroscopy is used with the patient in either general anesthesia, spinal block or even local anestesia. Standard instruments are used, available at any orthopaedic institution. Whether to use saline or gas arthroscopy is determined empirically. Both options are available. The area of interest on the femur is brought into vision by properly flexing the joint. Recesses, for example holes, corresponding to the shape of the primary fixation means on the implant, are machined, e.g. drilled, into the sub-chondral bone. Further, the remaining bone surface will be machined as to draw blood in order for adherence to the bioactive layer of the implant to occur. The implant is inserted into the joint and the fixation pegs are made to engage into the corresponding recesses. Subsequently, the implant is hammered in place. The thickness of the implant is such that it matches that of the surrounding cartilage. The edges are slightly undercut so as to become somewhat countersunk below the level of the surrounding cartilage. Initial fixation, herein also called primary fixation, of the implant occurs by interference fit between fixation pegs and the corresponding holes. Definite fixation, herein also called secondary fixation, is obtained by the bioactive layer bonding with host bone.
Method 2. With focal areas of degeneration, the transition to normal cartilage is gradual and not suitable for an implant as above. This will require a two-stage procedure. Here it is anticipated that a primary arthroscopy is made and some cartilage is burred away around the edges of the damaged area in order to create a situation as above. Subsequently, CT/MRI is performed and the procedure is then carried out as above.

For some joints, it is anticipated that the two stages in point 2 above can be made at one operative procedure where a cartilage defect is "created" through excision of worn edges of degenerated cartilage until stable and healthy cartilage is reached and the defect is created to fit an implant that is prefabricated to fit the underlying bone. In this case the operation will be performed as a small open, as opposed to scopic, procedure.

The implant of the present invention may be implanted or used on one side, tibial or femoral, of the knee or on both sides in the knee. In the latter case as two separate component, one on the tibial side and one on the femoral side.

### Further features

Production of the implant in accordance with invention are in different production methods made for individual fitting or in standard dimensions. In an individual fitting production method, the implant will be custom made for a particular joint of a particular patient in order for the curvature of the implant component to be identical to that of the sub-chondral bone plate at the site of insertion. In the alternative standard dimension variety, it is for example provided a kit of implant components of different sizes and shapes available, implying no need for individual tailoring.

In an individual fitting production method, the implant may be produced after drawings obtained after a CT scan or MRI of the specific part of the body intended for the implant. The tailored shape is obtained through production in a tailor made mould, or through post sintering treatment, including cutting, grinding and polishing or a mixture thereof.

The production method would in this embodiment comprise the steps of:
(i) performing a CT scan or MRI on the patient in order to determine the desired size and shape of the implant component;
(ii) creating graphite tools for the spark plasma sintering unit shaped according to CAD-drawings created according to results from the CT scan/MRI;

In a standard dimensions production method the implant is formed according to a standard size and shape, typically a cylindrical or slightly elongated component, and that the individual design is obtained through post compaction grinding of the gradient material according to drawings created for the specific patient.

The present invention may be provided in a kit which contains a selection of implants according to the invention, wherein the selection of implants comprises implants of different sizes and shapes. This means that an implant according to the invention, which is of suitable size and shape for the patient to be treated, is available for the surgeon who adjusts the excision of the cartilage to the available components.

## Claims

1. A medical implant (1) for replacing damaged cartilage in an articulating surface of a joint, comprising an implant body (2), designed as a thin plate or plate-like, possibly concave/convex, structure, having a first surface (5), a second surface (7) facing mutually opposite directions and a thickness therebetween;
the first surface (5) comprising a first biocompatible wear resistant metal, metal alloy or ceramic and being devised to form a wear resistant articulate surface configured to face the articulating part of the joint;
the second surface (7) comprising a bioactive ceramic or bioactive glass incorporated into a second metal, metal alloy or ceramic and being devised to form a bone contacting surface configured to face bone structure in the joint;
**characterized in that**:
the first surface (5) extends over an area of between 0,5 cm² and 10 cm² and the thickness between the first and the second surfaces (5, 7) is between 1 mm and 10 mm;
wherein the second surface (7) is a sintered mixtures comprising the bioactive ceramic or bioactive glass and the second metal, metal alloy or ceramic, the mixture having a homogenous microstructure; and
the first surface (5) and the second surface (7) are adhered by means of a sintered material structure.

2. The medical implant of claim 1, wherein the second surface comprises bioactive ceramic or bioactive glass in the range of 20-80 weight %.

3. The medical implant of any of the preceding claims, wherein said sintered material is achieved by electric pulse assisted consolidation (EPAC, SPS).

4. The medical implant of any of the preceding claims, wherein the sintered material structure adhering said first surface and said second surface is in the form of a functionally graded material providing a gradual change of mechanical, compositional and/or microstructural properties with position.

5. The medical implant of claim 4, wherein the functionally graded material comprises a binding structure that comprise metal material elements in common with the first metal, metal alloy or ceramic element of the first surface and with the second metal material elements in common with the second metal, metal alloy or ceramic of the second surface.

6. The medical implant of any of the preceding claims, wherein the first and/or the second metal, metal alloy or ceramic is any of stainless steel, cobalt-based alloys, chrome-based alloys, titanium-based alloys, pure titanium, zirconium-based alloys, tantalum, niobium, precious metals and their alloys, aluminium oxide, silicon nitride or yttria-stabilized zirconia, preferably stainless steel or a cobalt chrome alloy.

7. The medical implant of claim 6, wherein the first metal, metal alloy or ceramic is the same as the second metal, metal alloy or ceramic or wherein the first metal, metal alloy or ceramic is different from the second metal, metal alloy or ceramic.

8. The medical implant of any of the preceding claims, wherein the bioactive ceramic material is chosen from the group of hydroxyapatite, calcium sulphate, calcium phosphate, calcium aluminates, calcium silicates, calcium carbonates or combinations thereof, or bioactive glass, preferably hydroxyapatite.

9. The medical implant of any of the preceding claims, wherein
the first surface comprises stainless steel, and;
the second surface comprises a sintered material having a homogenous microstructure comprising a mixture of stainless steel or a cobalt chromium alloy and a bioactive ceramic material or bioactive glass.

10. The medical implant of any of claim 9, wherein
the first surface comprises stainless steel sintered from stainless steel raw material particles having a particle size which is less than 25 µm;
the second surface comprises a sintered material having a homogenous microstructure comprising a mixture of stainless steel or a cobalt chromium alloy and a bioactive ceramic material or bioactive glass;
the adherence between the first and the second surfaces is achieved by a porous layer configured between the first and the second surfaces comprising stainless steel sintered from stainless steel raw material particles having a particle size which is larger than 75 µm.

11. The medical implant of claim 1, wherein the adherence between the first and the second surfaces is achieved by a sintered material structure comprising
one or more layers configured between the first and the second surfaces, each layer comprising a sintered mixture having a homogenous microstructure of the biocompatible metal, metal alloy or ceramic and the bioactive ceramic or bioactive glass, wherein
the ratio of the bioactive ceramic material to stainless steel gradually changes over the layers, such that the layers comprise an increasing ratio of the bioactive ceramic material when going from the first surface towards the second surface layer.

12. The medical implant of any of the preceding claims, further comprising primary fixation means (4) for mechanical attachment to the bone, for example a screw, peg, keel or barb.

13. The medical implant of claim 12, wherein the primary fixation means (4) comprises the bioactive ceramic or bioactive glass of the second surface.

14. The medical implant of claim 12, wherein the primary fixation means (4) comprises the biocompatible metal, metal alloy or ceramic of the first surface, the primary fixation means (4) optionally being formes as a protrusion from a first surface layer (10) extending through a second surface layer (11) and possible intermediate layer or layers (12).

15. The medical implant of the preceding claim 14, wherein the primary fixation means (4) is formed as a protrusion from a first surface layer (10) extending through a second surface layer (11) and possible intermediate layer or layers (12).

## Patentansprüche

1. Medizinisches Implantat (1) zum Ersetzen beschädigten Knorpels in einer Gleitfläche eines Gelenks, umfassend
einen Implantatkörper (2), der als eine dünne Platte oder plattenähnliche, möglicherweise konkave/konvexe Struktur ausgelegt ist, mit einer ersten Oberfläche (5), einer zweiten Oberfläche (7), die in wechselseitig gegenüberliegende Richtungen weisen, und einer Dicke dazwischen;
wobei die erste Oberfläche (5) ein(e) erste(s) biokompatible(s) verschleißfeste(s) Metall, Metalllegierung oder Keramik umfasst, und konzipiert ist, eine verschleißfeste Gleitfläche zu bilden, die konfiguriert ist, dem gleitenden Teil des Gelenks gegenüberzuliegen;
wobei die zweite Oberfläche (7) eine bioaktive Keramik oder ein bioaktives Glas umfasst, das in ein(e) zweite(s) Metall, Metalllegierung oder Keramik eingebunden ist, und die konzipiert ist, eine Knochenkontaktfläche zu bilden, die konfiguriert ist, einer Knochenstruktur in dem Gelenk gegenüberzuliegen;
**dadurch gekennzeichnet, dass**
sich die erste Oberfläche (5) über einen Bereich zwischen 0,5 cm² und 10 cm² erstreckt und die Dicke zwischen der ersten und der zweiten Oberfläche (5, 7) zwischen 1 mm und 10 mm beträgt;
die zweite Oberfläche (7) eine gesinterte Mischung ist, die die bioaktive Keramik oder das bioaktive Glas und das zweite Metall, die zweite Metall-Legierung oder die zweite Keramik umfasst, wobei die Mischung eine homogene Mikrostruktur aufweist; und die erste Oberfläche (5) und die zweite Oberfläche (7) mittels einer gesinterten Materialstruktur miteinander verhaftet sind.

2. Medizinisches Implantat gemäß Anspruch 1, wobei die zweite Oberfläche bioaktive Keramik oder bioaktives Glas in dem Bereich von 20-80 Gew.-% umfasst.

3. Medizinisches Implantat gemäß einem der vorstehenden Ansprüche, wobei das gesinterte Material durch Elektropuls-unterstütztes Verfestigen (EPAC, SPS) erhalten wird.

4. Medizinisches Implantat gemäß einem der vorstehenden Ansprüche, wobei die gesinterte Materialstruktur, die an der ersten Oberfläche und der zweiten Oberfläche haftet, in der Form eines funktionell gradierten Materials vorliegt, das eine mit der Position gradierte Veränderung von mechanischen, Zusammensetzungs- und/oder mikrostrukturellen Eigenschaften bereitstellt.

5. Medizinisches Implantat gemäß Anspruch 4, wobei das funktionell gradierte Material eine Bindungsstruktur umfasst, die Metallmaterialelemente umfasst, die sie mit dem ersten Metall, der ersten Metall-Legierung oder dem ersten Keramik-Element der ersten Oberfläche gemein hat, und mit den zweiten Metallmaterialelementen, die sie mit dem zweiten Metall, der zweiten Metall-Legierung oder der zweiten Keramik der zweiten Oberfläche gemein hat.

6. Medizinisches Implantat gemäß einem der vorstehenden Ansprüche, wobei das erste und/oder das zweite Metall, die erste und/oder die zweite Metall-Legierung oder die erste und/oder die zweite Keramik eine von Edelstahl, Kobalt basierte Legierungen, Chrom basierte Legierungen, Titan basierte Legierungen, reines Titan, Zirkon basierte Legierungen, Tantal, Niob, Edelmetalle und deren Legierungen, Aluminiumoxid, Siliziumnitrid oder Yttriumoxid stabilisiertes Zirconium, vorzugsweise Edelstahl oder eine Kobalt-Chrom-Legierung ist.

7. Medizinisches Implantat gemäß Anspruch 6, wobei das erste Metall, die erste Metall-Legierung oder die erste Keramik gleich dem zweiten Metall, der zweiten Metall-Legierung oder der zweiten Keramik ist, oder wobei sich das erste Metall, die erste Metall-Legierung oder die erste Keramik von dem zweiten Metall, der zweiten Metall-Legierung oder der zweiten Keramik unterscheidet.

8. Medizinisches Implantat gemäß einem der vorstehenden Ansprüche, wobei das bioaktive Keramikmaterial ausgewählt ist aus der Gruppe von Hydroxylapatit, Calciumsulfat, Calciumphosphat, Calciumaluminate, Calciumsilikate, Calciumcarbonate oder Kombinationen davon oder bioaktives Glas, vorzugsweise Hydroxylapatit.

9. Medizinisches Implantat gemäß einem der vorstehenden Ansprüche, wobei die erste Oberfläche Edelstahl umfasst; und
die zweite Oberfläche ein gesintertes Material mit einer homogenen Mikrostruktur umfasst, die eine Mischung aus Edelstahl oder einer Kobalt-Chrom-Legierung und einem bioaktiven Keramikmaterial oder einem bioaktiven Glas umfasst.

10. Medizinisches Implantat gemäß einem Anspruch 9, wobei
die erste Oberfläche Edelstahl umfasst, der aus Edelstahl-Rohmaterial-Partikeln gesintert ist, die eine Partikelgröße aufweisen, die kleiner als 25 µm ist;
die zweite Oberfläche ein gesintertes Material mit einer homogenen Mikrostruktur umfasst, die eine Mischung aus Edelstahl oder einer Kobalt-Chrom-Legierung und einem bioaktiven Keramikmaterial oder einem bioaktiven Glas umfasst;
die Haftung zwischen der ersten und der zweiten Oberfläche durch eine poröse Lage erreicht wird, die zwischen der ersten und der zweiten Oberfläche konfiguriert ist und die Edelstahl umfasst, der aus Edelstahl-Rohmaterial-Partikeln gesintert ist, die eine Partikelgröße aufweisen, die größer als 75 µm ist.

11. Medizinisches Implantat gemäß Anspruch 1, wobei
die Haftung zwischen der ersten und der zweiten Oberfläche durch eine gesinterte Materialstruktur erreicht wird, umfassend:
eine oder mehrere Lagen, die zwischen der ersten und der zweiten Oberfläche konfiguriert sind, und wobei jede Lage eine gesinterte Mischung ist, die eine homogene Mikrostruktur des biokompatiblen Metalls, der biokompatiblen Metall-Legierung oder der biokompatiblen Keramik und der bioaktiven Keramik oder dem bioaktiven Glas aufweist,
wobei das Verhältnis des bioaktiven Keramikmaterials zu dem Edelstahl sich graduell über die Lagen ändert, sodass die Lagen ein ansteigendes Verhältnis des bioaktiven Keramikmaterials beim Übergang von der ersten Oberfläche zu der zweiten Oberflächenlage umfassen.

12. Medizinisches Implantat gemäß einem der vorstehenden Ansprüche, weiter umfassend primäre Befestigungsmittel (4) zur mechanischen Anbringung an dem Knochen, beispielsweise eine Schraube, ein Stift, ein Kiel oder ein Stachel.

13. Medizinisches Implantat gemäß Anspruch 12, wobei das primäre Befestigungsmittel (4) die bioaktive Keramik oder das bioaktive Glas der zweiten Oberfläche umfasst.

14. Medizinisches Implantat gemäß Anspruch 12, wobei das primäre Befestigungsmittel (4) das biokompatible Metall, die biokompatible Metall-Legierung oder die biokompatible Keramik der ersten Oberfläche umfasst, wobei das primäre Befestigungsmittel (4) optional als ein Vorsprung von einer ersten Oberflächenlage (10) geformt ist, die sich durch eine zweite Oberflächenlage (11) und eine oder mehrere mögliche Zwischenlagen (12) erstreckt.

15. Medizinisches Implantat gemäß Anspruch 14, wobei das primäre Befestigungsmittel (4) als ein Vorsprung von einer ersten Oberflächenlage (10) geformt ist, die sich durch eine zweite Oberflächenlage (11) und eine oder mehrere mögliche Zwischenlagen (12) erstreckt.

## Revendications

1. Un implant médical (1) pour le remplacement du cartilage endommagé dans une surface d'articulation d'une articulation, comprenant un corps d'implant (2), réalisé sous la forme d'une structure en plaque mince ou assimilable à une plaque, éventuellement concave/convexe, ayant une première surface (5) et une deuxième surface (7) tournées selon des directions mutuellement opposées, et ayant une épaisseur entre celles-ci,
la première surface (5) comprenant un premier métal biocompatible résistant à l'usure, un alliage métallique, ou une céramique et étant conçue pour former une surface d'articulation résistante à l'usure, configurée pour faire face à la pièce d'articulation de articulation ;
la deuxième surface (7) comprenant une céramique bioactive ou un verre bioactif incorporé dans un second métal, un alliage métallique ou une céramique, et étant conçue pour former une surface de contact avec l'os, configurée pour faire face à la structure osseuse de l'articulation ;
**caractérisé en ce que** :
la première surface (5) s'étend sur une aire comprise entre 0,5 cm² et 10 cm² et l'épaisseur entre la première et la deuxième surfaces (5, 7) est comprise entre 1 mm et 10 mm ;
la deuxième surface (7) est un mélange fritté comprenant la céramique bioactive ou le verre bioactif et le second métal, l'alliage métallique ou la céramique, le mélange ayant une microstructure homogène, et
la première surface (5) et la deuxième surface (7) sont solidarisées au moyen d'une structure en matériau fritté.

2. L'implant médical selon la revendication 1, dans lequel la deuxième surface comprend une céramique bioactive ou un verre bioactif dans la plage allant de 20 à 80% en poids.

3. L'implant médical selon l'une quelconque des revendications précédentes, dans lequel ledit matériau fritté est obtenu par consolidation assistée par des impulsions électriques (EPAC, SPS).

4. L'implant médical selon l'une quelconque des revendications précédentes, dans lequel la structure de matériau fritté réalisant l'adhésion de ladite première surface à ladite deuxième surface est sous la forme d'un matériau à gradation fonctionnelle fournissant un changement graduel de propriétés mécaniques, de composition et / ou de microstructure selon sa position.

5. L'implant médical selon la revendication 4, dans lequel le matériau à gradation fonctionnelle comprend une structure de liaison qui comporte des éléments de matériau métallique communs avec l'élément en premier métal, alliage métallique ou céramique de la première surface et avec des éléments de matière du second métal communs avec le second métal, alliage métallique ou céramique de la deuxième surface.

6. L'implant médical selon l'une quelconque des revendications précédentes, dans lequel le premier et / ou le second métal, alliage métallique ou céramique est l'un parmi les matériaux suivants : acier inoxydable, alliages à base de cobalt, alliages à base de chrome, alliages à base de titane, titane pur, alliages à base de zirconium, tantale, niobium, métaux précieux et leurs alliages, oxyde d'aluminium, nitrure de silicium ou zirconium stabilisé d'yttrium, et de préférence en acier inoxydable ou en un alliage chrome-cobalt.

7. L'implant médical selon la revendication 6, dans lequel le premier métal, alliage métallique ou céramique est le même que le second métal, alliage métallique ou céramique, ou dans lequel le premier métal, alliage métallique ou céramique est différent du second métal, alliage métallique ou céramique.

8. L'implant médical selon l'une quelconque des revendications précédentes, dans lequel le matériau céramique bioactif est choisi dans le groupe comprenant l'hydroxyapatite, le sulfate de calcium, le phosphate de calcium, les aluminates de calcium, les silicates de calcium, les carbonates de calcium ou des combinaisons de ceux-ci, ou du verre bioactif, et de préférence l'hydroxyapatite.

9. L'implant médical selon l'une quelconque des revendications précédentes, dans lequel
la première surface comprend de l'acier inoxydable, et
la deuxième surface comprend un matériau fritté ayant une microstructure homogène, comprenant un mélange d'acier inoxydable ou d'un alliage de chrome-cobalt et d'un matériau céramique bioactif ou du verre bioactif.

10. L'implant médical selon la revendication 9, dans lequel
la première surface comprend de l'acier inoxydable obtenu par frittage de particules de matière brute d'acier inoxydable ayant une taille de particule qui est inférieure à 25 µm ;
la deuxième surface comprend un matériau fritté ayant une microstructure homogène, comprenant un mélange d'acier inoxydable ou d'un alliage de chrome-cobalt et un matériau céramique bioactif ou du verre bioactif ;
l'adhérence entre les première et deuxième surfaces est réalisée par une couche poreuse s'étendant entre les première et deuxième surfaces comprenant de l'acier inoxydable obtenu par frittage de particules de matière brute d'acier inoxydable ayant une taille de particule qui est supérieure à 75 µm.

11. L'implant médical selon la revendication 1, dans lequel l'adhérence entre les première et deuxième surfaces est réalisée par une structure de matériau fritté comprenant
une ou plusieurs couches s'étendant entre les première et deuxième surfaces, chaque couche comprenant un mélange fritté ayant une microstructure homogène du métal biocompatible, de l'alliage métallique ou de la céramique et de la céramique bioactive ou du verre bioactif,
le rapport entre le matériau céramique bioactif et l'acier inoxydable changeant progressivement le long des couches, de telle sorte que les couches comprennent un rapport de matériau céramique bioactif allant en augmentant en allant de la première surface vers la couche de deuxième surface.

12. L'implant médical selon l'une quelconque des revendications précédentes, comprenant en outre des moyens de fixation primaires (4) pour la fixation mécanique à l'os, par exemple une vis, un tenon, une quille ou un ardillon.

13. L'implant médical selon la revendication 12, dans lequel les moyens de fixation primaires (4) comprennent la céramique bioactive ou le verre bioactif de la deuxième surface.

14. L'implant médical selon la revendication 12, dans lequel les moyens de fixation primaires (4) comprennent le métal, l'alliage métallique ou la céramique biocompatible de la première surface, les moyens de fixation primaires (4) étant optionnellement réalisés sous la forme d'une saillie se projetant d'une première couche de surface (10), s'étendant à travers une seconde couche de surface (11) et éventuellement à travers une ou plusieurs couches intermédiaires (12).

15. L'implant médical selon la revendication 14 qui précède, dans lequel les moyens de fixation primaire (4) sont réalisés sous la forme d'une saillie se projetant d'une première couche de surface (10), s'étendant à travers une seconde couche de surface (11) et, éventuellement, à travers une ou plusieurs couches intermédiaires (12).
